# EUROPEAN PATENT APPLICATION

(11) **EP 0 578 472 A2**
(43) Date of publication of application: **12.01.1994**
(21) Application number: 93305282.1
(22) Date of filing: 06.07.1993
(51) Int. Cl.: C12N 15/62, C07K 1/12, C12P 21/02, C12N 15/16, A61K 37/02

(54) **A process for recovering peptides expressed as fusion proteins**

(30) Priority: 07.07.1992 JP 179713/92
(71) Applicant: SANKYO COMPANY LIMITED, Chuo-ku, Tokyo 103 (JP)
(72) Inventor: Tamaoki, Hidetsune, c/o Sankyo Company Limited, Iwaki-shi, Fukushima-ken (JP); Yoshikawa, Hiroji, c/o Sankyo Company Limited, Iwaki-shi, Fukushima-ken (JP); Ishikawa, Hirokazu, c/o Sankyo Company Limited, Shinagawa-ku, Tokyo 140 (JP); Kawaguchi, Junko, c/o Sankyo Company Limited, Shinagawa-ku, Tokyo 140 (JP); Kaneko, Isao, c/o Sankyo Company Limited, Shinagawa-ku, Tokyo 140 (JP)
(74) Representative: Gibson, Christian John Robert

(57) **Abstract**

A process for recovering peptides which are expressed as fusion proteins comprises solubilising the fusion protein and subjecting it to proteolytic cleavage, the improvement comprising citraconylating free amino groups prior to solubilisation, thereby both to enhance solubility of the fusion protein in conditions conducive to proteolytic cleavage and to improve yield. It is also advantageous to sulphonate any disulphide bridges present in the fusion protein prior to cleavage.

## Description

The present invention relates to a process for obtaining a peptide (the target peptide) from a fusion protein of which the peptide forms a part, the process comprising the steps of solubilising the fusion protein and then subjecting the solubilised protein to proteolytic cleavage to yield the target peptide.

Many naturally occurring substances are useful, or are potentially useful, in many fields of industry, but those which are of use in medicine often occur only in small quantities in nature, or are produced under conditions requiring stringent quality control. A typical family of such substances is the family of lymphokines, or interferons, which are naturally produced only in extremely small amounts, and only when the natural source has been stimulated, generally by a pathogen, such as a virus. Purification of such small amounts of interferon, in this instance, is difficult in itself, but the task is further complicated by having to ensure that the resulting preparation is also pathogen-free.

Genetic engineering seeks to provide the solution to this problem, among others. If the genetic material encoding the desired substance can be isolated, then it can be introduced, by suitable manipulation, into an alternative cell system to allow the substance to be produced in useful quantities and without any adverse contamination.

Suitable cell systems may be either prokaryotic or eukaryotic, but prokaryotic systems are generally preferred because of their ease of manipulation, including transformation with foreign genetic material, and cultivation.

Although it easier to use prokaryotic systems, there is often a further problem. This problem arises from the fact that many of the substances which it is desired to produce by genetic engineering are of eukaryotic origin. Eukaryotic and prokaryotic cells employ similar, but not identical, pathways in the synthesis of proteins, for example, but the end-product from a prokaryotic cell is frequently not biologically active when the encoding genetic material is eukaryotic in origin.

One reason for prokaryotic cells producing inactive equivalents of eukaryotic proteins is that post-translational modification, such as amidating the C-terminal amino acid (e.g. proline), glycosylation, or processing of proenzymes, is common in eukaryotic cells, but that the necessary mechanisms are not present in prokaryotic cells.

It is also often the case that large amounts of the desired substance, whether active or inactive, are synthesised by the prokaryotic microorganism, but only a small amount can be isolated. This can happen because the substance is unstable in the cellular environment, or is being digested because it is heterologous and, therefore, provides a target for digestive enzymes. Substances such as low molecular weight peptides are most susceptible to this problem.

To overcome this problem, small peptides can be expressed as a part of a fusion protein. The expression vector encodes a polypeptide chain of which the desired, or target, peptide forms a part. The resulting polypeptide, or fusion protein, is expressed by the microorganism in a form which is stable and which is not digested, frequently in the form of insoluble inclusion bodies. The inclusion bodies can then be harvested by disrupting the cells and collecting the inclusion bodies by centrifugation.

To obtain the target peptide from the fusion protein, it is necessary to digest the fusion protein, such as by using a specific protease, to cleave off the remainder of the fusion protein (secondary peptide) and leave only the target peptide. To permit action of the protease, the fusion protein must be brought into solution. However, the conditions necessary to solubilise fusion proteins frequently require very high concentrations of, for example, urea as solubilising agents, thereby substantially inhibiting the action of the protease. Any reduction in urea concentration reduces the amount of fusion protein solubilised and ultimately, at a level where the protease is able to act, maximum final yields of around 30% are all that is possible.

More specifically, an example of a peptide which, when made by genetic engineering, is produced as a fusion protein, is calcitonin. Calcitonin is a hormone which governs calcium metabolism. It lowers the concentration of calcium in the blood by suppressing bone resorption, which is caused primarily by osteoclasts. The efficacy of calcitonin as a therapeutic drug in the treatment of osteoporosis, Paget's disease and hypercalcemia is extremely high.

Calcitonin is a peptide consisting of 32 amino acids, the α-carboxyl group of the C-terminal proline being amidated. It is produced in the thyroid of mammals and in the ultimobranchial gland in birds and fish. The fact that it is only present in small amounts in these glands has meant that it has been impossible to collect large amounts of naturally occurring calcitonin from tissues. However, recent progress in the field of genetic engineering has overcome this problem, so that calcitonin can now be produced in large quantities as a recombinant peptide.

As mentioned briefly above, suitable host-vector systems for producing recombinant peptides include those based on bacterial cells, animal cells and yeast cells but, when production on an industrial scale is intended, Escherichia coli is normally used, since this is a system that allows expression of the target substance in large amounts, relatively easily. The gene encoding calcitonin is inserted into a suitable expression vector, and calcitonin is then expressed and can be collected by culturing the transformed host.

As calcitonin has a relatively low molecular weight, the expression vector is constructed to express a fusion protein, wherein calcitonin is fused with another peptide, so that the expression product has a stable molecular structure. Calcitonin can then be obtained by digestion of the expressed fusion protein.

In order to increase the amount of target protein, it is important to select a host-vector system that allows efficient expression, and to choose culture conditions that will maximise production. It is particularly important for the target peptide to be efficiently recovered from the resulting fusion protein.

When Escherichia coli is used as a host-vector system for the expression of fusion proteins, it is generally the case that the fusion proteins accumulate in insoluble form in inclusion bodies within the bacterium. As stated above, the fact that the fusion protein is obtained as an insoluble substance has the advantage that purification is made easy. After the cells have been homogenised, high-purity fusion protein can be obtained from the the insoluble fraction simply by centrifugation and washing with a suitable buffer containing a non-ionic detergent, such as Triton (trade mark) X-100.

To subsequently obtain calcitonin, the purified fusion protein must first be solubilised to allow its use in the cleavage reaction.

Examples of solubilising agents often used include highly concentrated aqueous solutions of guanidine hydrochloride (5 to 6 M) and urea (8 to 10 M). However, proteases used in the cleavage reactions are inactive at such high concentrations of solubilising agents. Consequently, lower concentrations of the solubilising agents must be used (1 to 2 M, in the case of guanidine hydrochloride, and 3 to 4 M in the case of urea). A significant fraction of the fusion protein then remains insoluble, with a concomitant and sizeable reduction in yield.

The overall process has typically been carried out, to date, using the following procedure.

Escherichia coli, which has been transformed with an expression vector encoding a fusion protein of which human calcitonin peptide forms a part, is cultured and then homogenised. The fusion protein expressed is in the form of an insoluble fraction, which is harvested and then dissolved in 0.05 M Tris [tris(hydroxymethyl)-aminomethane] acetic acid buffer (pH 7.5) containing 10 M urea. The cleavage reaction can then be performed using clostripain, a clostridial protease, after the resulting solution has been diluted with a similar buffer, but containing β-mercaptoethanol (as an enzyme stabiliser) and calcium nitrate, reducing the urea concentration to 3.3 M. The enzyme (0.25 U/mg substrate) is then allowed to act at 37°C for 3 hours.

This procedure results in a yield of around 30% calcitonin, relative to the total amount of fusion protein. Such a yield is low. Extending the reaction time and using additional clostripain, however, does not result in a significantly improved yield.

Sodium dodecyl sulphate polyacrylamide gel electrophoresis and densitometric analysis, when performed on the preparation of fusion protein immediately prior to the cleavage reaction, and after removal of insolubles from the preparation by centrifugation, demonstrate that soluble fusion protein represents only about one third of the total fusion protein, i.e. two thirds of the total protein remains insoluble. Given that the yield of calcitonin is about 30%, it would appear extremely likely that only fusion protein in the solubilised state can serve as a substrate for the enzyme, and that the enzyme can act only slightly or not at all on fusion protein which has not been solubilised.

Thus, it will be appreciated that the cleavage reaction and conditions affecting the reaction are extremely important in the production of peptides which are initially expressed as fusion proteins. It is essential for the fusion protein to be in solution, or solubilised, for the reaction to take place, but full solubilisation of the fusion protein can only be achieved at the expense of enzyme activity.

Accordingly, there is a need to find a method for enhancing the solubility of fusion proteins without deleteriously affecting protease activity. The problem is that, while chemical modification can produce the desired degree of solubility, the resulting peptide achieved after protease cleavage must retain biological activity to be useful, so that it is not possible to effect major alterations to the chemical structure of the fusion protein. Effective solvents have been sought but, as shown above, when the solvent is sufficiently effective to dissolve all of the fusion protein, enzyme activity is prevented.

We have now discovered that it is possible to dissolve fusion proteins effectively completely in such a manner that proteases are still able to act. We have found that acidic groups can be reversibly introduced into the side chains of the fusion peptide, and that this makes the protein remarkably more soluble. It is likely that this is because the pH during solubilisation and cleavage is in the neutral or weakly alkaline range.

We have further discovered that this effect can be improved by preventing or disrupting disulphide bridge formation, both intermolecular and intramolecular, by reversibly derivatising disulphide bonds.

Thus, in a first aspect, there is provided a process for recovering a peptide from a fusion protein incorporating the peptide, comprising the steps of:
i) at least partially solubilising the fusion protein,
ii) subjecting the solubilised fusion protein to proteolytic cleavage and,
iii) isolating the peptide,

characterised in that step i) comprises citraconylating free amino groups of the fusion protein.

It is further preferred that, where appropriate, step i) additionally comprises sulphonating free -SH groups or disulphide linkages of the fusion protein.

Citraconylation of the amino groups entails the introduction of a citraconyl moiety at the amino group. Citraconylation can be effected by any suitable means known in the art, such as by reacting the fusion protein with citraconic acid in the presence of a condensing agent, reaction with an appropriate acid halide, or reaction with citraconic anhydride. Where a condensing agent is used, this is suitably carbonyldiimidazole or dicyclohexylcarbodiimide. Where an acid halide is employed, the chloride is preferred. However, it is particularly preferred to use citraconic anhydride. Where citraconylation is referred to herein, it will generally be described as being achieved by using citraconic anhydride, but it will be appreciated that any other suitable means of citraconylation are also envisaged, such as those described above.

Citraconylating with citraconic anhydride is a reversible process, and the amino groups affected are generally the ε-amino groups of lysine residues and the N-terminal α-amino group. Derivatisation with citraconic anhydride may be effected directly by exposure of the fusion protein to citraconic anhydride under appropriate conditions, provided that the end result is to citraconylate amino groups. A suitable citraconylation process is described by Dixon, H.B.F., et al., in Biochem. J. (1968), 109, 312.

Direct exposure to citraconic anhydride is the preferred means for achieving citraconylation, and it will be appreciated that the reaction will generally be quicker if at least some of the fusion protein is already in solution. Accordingly, it is preferred to treat the preparation of fusion protein with a known solubilising agent, such as concentrated urea or guanidine hydrochloride, prior to conducting the citraconylation reaction. Once amino groups of the fusion protein have been citraconylated to the desired extent, typically in the region of 95%, the reaction solution can be diluted to lower the concentrations of the other solubilising agents thereby to permit enzyme activity.

Reversible S-sulphonation protects, for example, the SH groups of cysteine residues by cleaving the disulphide bond. A suitable method is described by David, R. Cole, in Methods in Enzymology (1967), Ed. Hirs, C.H.W., vol. XI, pp. 206, Academic Press, New York and London.

As with citraconylation, the step of S-sulphonation is preferably conducted at least partially in solution for best results. Again, it is not essential that the fusion protein be pre-solubilised to any extent, but it is generally beneficial to do so.

S-Sulphonation can be performed before or after citraconylation, if it is desired to employ this step. In general, it is preferred to sulphonate after citraconylation, as the reaction tends to be most effective for proteins already solubilised to some degree.

While the step of solubilisation may merely comprise citraconylating the fusion protein to bring it into solution, it is preferred that the protein is also S-sulphonated. It is particularly preferred that citraconylation and, where desired, S-sulphonation are used in conjunction with known solubilising agents/ techniques in order to achieve maximal solubilisation.

Once the target peptide has been harvested, after the cleavage reaction, it will generally be preferable to remove the citraconyl groups and, where appropriate, the sulphonate groups. Both reactions are described in more detail hereinbelow, for preferred embodiments. In general, the deprotection reactions are straightforward, and decitraconylation, for example, can often be effected merely by letting the reaction solution stand, given that protease reactions will generally be stopped by acidification of the solution, acidic conditions being useful to cause decitraconylation.

The proteolytic cleavage step may be performed in any suitable manner, such as is described hereinbelow, but it is preferred to use a protease. Suitable proteases will be readily apparent to those skilled in the art. In general, proteases will be selected according to the nature of the fusion protein, appropriate enzymes serving to cleave the protein at a chosen point, usually as near as possible to the junction of target peptide and secondary peptide without also serving to cleave any portion of the target peptide. We particularly prefer to use trypsin, owing to its ready availability and low cost.

In a preferred embodiment, the present invention provides a process for preparing calcitonin comprising:
(1) culturing Escherichia coli transformed by an expression vector encoding a calcitonin peptide fusion protein, and collecting insoluble material containing the fusion protein after homogenisation of the culture,
(2) citraconylating and optionally S-sulphonating the fusion protein contained in the insoluble fraction to solubilise the protein, and
(3) proteolytically cleaving the fusion protein to remove non-calcitonin peptide from the soluble protein, and collecting the resulting calcitonin.

Thus, the present invention provides an improved process for preparing a target peptide, such as calcitonin, which comprises culturing a host, such as Escherichia coli, transformed by an expression vector for the expression of a fusion protein wherein one or more other peptides or proteins (secondary peptides) are linked to the target peptide, the fusion protein being expressed as an insoluble material, solubilising the resulting insoluble fusion protein, and removing the non-target peptide parts from the fusion protein, preferably using a protease, and collecting the target peptide.

By using a process according to the present invention, yields from the cleavage reaction are generally 2 to 3 times greater than those obtained in the prior art, primarily because the protease, we believe, is able to act effectively on total fusion protein, rather than just a fraction.

Furthermore, where a process according to the present invention is used to produce human or rat calcitonin, for example, it is possible to employ trypsin as the protease. Thus, not only does the present invention have the advantage of improving yields, it can also lead to a reduction in costs, by allowing the use of trypsin, which is relatively cheap and readily available.

Where the present invention is used to obtain calcitonin, the calcitonin in question may be from any suitable origin, such as human, chicken, pig, cow, sheep, rat, eel and salmon calcitonins [c.f. The Bone (1992), 3, vol. 8, No. 1, p. 30]. There is no limit on types of peptide which can be obtained in accordance with the present invention, but the invention is most applicable to the production of peptides which are expressed as insoluble fusion proteins. Even where calcitonin is the target, any number of variations on the nature of the calcitonin are possible, such as where one or more of the amino acid residues are substituted, inserted or deleted. It is preferred that the substance obtained still has the properties of the original, where calcitonin is prepared, for example, although this is not an essential requirement. Other modifications and suitable peptides for use in the processes of the present invention will be clear to those skilled in the art.

The preferred target peptide is human calcitonin.

In order to obtain biologically active calcitonin using recombinant techniques, it is necessary to artificially amidate the C-terminal proline α-carboxyl group, owing to the fact that Escherichia coli does not possess the necessary mechanism. Amidation can be performed by any suitable process. However, it is preferred that the peptide obtained after proteolytic cleavage is engineered such that it can be acted on by an enzyme to amidate the C-terminal carboxyl group. A suitable C-terminal amidation method for calcitonin employs yeast carboxypeptidase Y [Japanese Unexamined Patent Publication No. Sho-62-29997; and Tamaoki, H. et al., Ann. Rep. Sankyo Res. Lab., 38, 73C (1986)].

The preferred peptide substrate for carboxypeptidase Y has a leucine residue attached to a C-terminal proline. In the case of calcitonin-Leu (CT-Leu), digestion of the leucine residue leaves an amidated C-terminal proline. The amidation step is not an essential feature of the present invention, but is particularly preferred where amidation is necessary to obtain biological activity.

Selection and culture of the preferred Escherichia coli host-vector system may suitably be performed in accordance with known methods [c.f. Proteins, Nucleic Acids and Enzymes (1983), 28, No. 14].

The secondary component of the fusion protein, i.e. that which is attached to the target peptide, for example, is preferably homologous, or host-originated. Where Escherichia coli is the host, for example, it is preferred to use Escherichia coli derived proteins, such as β-galactosidase, chloramphenicol acetyl transferase (CAT) or maltose-binding protein, but proteins from other sources, such as Staphylococcal Protein A, may also be used, if desired. Particularly preferred examples of secondary peptide components are derived from β-galactosidase and chloramphenicol acetyl transferase.

It is generally preferred to have only one secondary peptide component in the fusion protein, but the present invention also envisages the use of two or more secondary components, as desired. The secondary peptides may be linked to either the N-terminal or C-terminal of the target peptide, there being no particular limitation, and there may be a secondary peptide attached to each end of the target peptide.

The fusion protein, in addition to the target peptide and secondary component, may also comprise other amino acid residues, such as may be required, for example, to provide a site for protease activity, or which may merely be artifacts of the genetic engineering method used to construct the expression vector.

Where Escherichia coli is employed as the host, any suitable expression vector can be used. A great many such vectors are well known, and we prefer to use pBR 322, PUC 18 or pDR 720. Any suitable strain of Escherichia coli can be used, provided that it is able to express the relevant fusion protein. Preferred strains include HB101, RB791, JM109, JA221 and DH5.

To obtain the target peptide from the fusion protein, chemical cleavage methods, such as the cyanogen bromide degradation method, which involves peptide cleavage at the α-carboxyl side of methionine residues [Gross, E. & Witkop, B., J. Biol Chem. (1962), 237, 1856], or peptide digestion methods using a protease, may be used. As these methods cleave at specific sites within a peptide, it is necessary to provide such a site by the appropriate genetic engineering methods, where a suitable site does not already exist. Thus, amino acids or peptide sequences may be inserted as linker portions between the target peptide and the secondary peptide, to provide the necessary cleavage site. The linker portion may completely embody the cleavage site, or may form a cleavage site in conjunction with one of the peptide portions of the fusion protein.

It will be appreciated that modification of the fusion protein to permit cleavage should take into consideration the requirement to obtain an integral end-product. If the cleavage reaction is such as to remove a part of the target peptide, then it would usually be inconvenient to have to reconstitute the peptide. Thus, it is strongly preferred to engineer the fusion protein in such a way that one simple cleavage reaction will yield the target peptide. From this point of view, the most preferred cleavage methods are those using clostripain (which cleaves only the peptide bond at the α-carboxyl side of arginine residues) and Staphylococcus aureus V8 protease (which cleaves only the peptide bond at the α-carboxyl side of glutamate residues). Other cleavage reactions are also preferred which leave a small number of residues, preferably one, attached to the target protein and which can be readily removed, such as by an exopeptidase. Again, carboxypeptidase Y can be used as an exopeptidase in this context.

These methods are not necessarily applicable to all fusion proteins, as some proteases may cleave some target peptides, and so should be selected with care. For example, salmon and eel calcitonins have an arginine residue at position 24, so that trypsin and clostripain cannot be used to digest fusion proteins containing these calcitonins, as the calcitonin would also be cleaved. Likewise, eel and chicken calcitonin peptides have a glutamic acid residue at position 15, as do chicken, cow, pig and sheep calcitonin peptides at position 30, so that Staphylococcus aureus V8 protease cannot be used, for a similar reason.

As used herein, the term "peptide" relates generally to any sequence of two or more amino acid residues which, in the case of the target peptide, it is desired, or convenient, to obtain as a fusion protein. Calcitonin, especially human calcitonin, is the preferred target peptide.

The following provides an example of a calcitonin peptide fusion protein.

On the assumption that clostripain is to be used for the cleavage reaction, a gene encoding the fusion protein CAT-Lys-Arg-hCT-Leu is constructed. This gene consists of a combination of the chloramphenicol acetyl transferase (CAT) gene, a codon pair encoding lysylarginine (Lys-Arg) as the linker peptide to provide a site for clostripain cleavage, and a gene encoding human calcitonin-Leu (hCT-Leu). The resulting fusion protein is effectively human calcitonin fused with bacterial chloramphenicol acetyl transferase. In order to align the nucleotide sequence reading frame, it is necessary to delete a nucleotide sequence encoding a portion of the C-terminal peptide of chloramphenicol acetyl transferase.

An expression vector containing the engineered gene is then constructed under the control, for example, of a Trp promoter, and any other suitable features which may assist in expression, control, replication and the like. The resulting vector is then used to transform a suitable host, such as Escherichia coli. The transformed host can then be cultured and the fusion protein harvested.

The molecular weight of a typical fusion protein obtained by this technique is in the region of 29 340, of which 3 520 is attributable to hCT-Leu. The number of free amino groups contained in a single molecule of this fusion protein (calculated on N-terminal α-amino groups and lysine ε-amino groups) is 15, including that of the lysine residue at position 17 of hCT-Leu. The total number of free SH groups is 7, including those of the cysteine residues at positions 1 and 7 of hCT-Leu.

A preferred example of a process according to the present invention, in this case to produce human calcitonin, is described below.

### 1. Preparation of Fusion Protein

A culture solution of Escherichia coli, transformed as described above, is collected by centrifugation and then homogenised using a homogeniser (Gorlin 15 M, APV Co.). The homogenised bacteria are washed by the addition of 5 volumes of 0.05 M Tris acetic acid buffer (pH 7.5) containing 0.5% v/v Triton X-100, and the resulting mixture is recentrifuged. The supernatant fraction is then discarded and the insoluble fraction containing the fusion protein is collected. A concentrated slurry (total protein concentration: 40 to 60 mg/ml) is obtained. The purity of the fusion protein in this state is approximately 30%.

### 2. Citraconylation

Citraconylation of the fusion protein is performed by adding citraconic anhydride to the concentrated slurry obtained in step 1, after addition of a solubilising agent (see below).

Prior to addition of citraconic anhydride, two volumes of 10 M urea solution are added to the concentrated slurry obtained in step 1, and the pH adjusted to 8.7 by the addition of a 5 N aqueous solution of sodium hydroxide. It is frequently the case that the fusion protein does not completely dissolve when only urea is added as a solubilising agent.

Aliquots of citraconic anhydride of about 250 µl per 1,000 mg of total protein are then added to the slurry at ambient temperature, with stirring. The time interval between additions is advantageously about 10 to 15 minutes. The pH of the mixture is maintained at 8.7 using 5 N aqueous sodium hydroxide.

A suitable amount of citraconic anhydride is 20 to 30 moles per 1 mole of free amino groups in the protein. If a greater amount of citraconic anhydride is used, then the risk of adverse side reactions becomes unfavorably large.

When all of the citraconic anhydride has been added, the fusion protein is in solubilised form, with 95% or more of the free amino groups in the protein being citraconylated.

Confirmation of the extent of citraconylation can be established by using 2,4,6-trinitrobenzene sulphonic acid (TNBS). TNBS binds free amino groups, so that the method described below provides an inverse indication of citraconylation.

500 µl of each of distilled water, 0.1 M aqueous sodium hydrogencarbonate, and 0.1% w/v aqueous TNBS are added to 15 to 30 µl of the solution containing the citraconylated fusion protein. The resulting mixture is left to stand at 40°C for 2 hours in the dark. Subsequently, 500 µl of 10% w/v aqueous sodium dodecyl sulphate and 250 µl of 1 N aqueous hydrochloric acid are added to the reaction mixture. Free amino groups which have not been citraconylated can then be measured by colorimetry at a wavelength of 335 nm using a blank solution of distilled water as a control.

In order to remove the citraconyl groups from the protein, 95% or more can be removed and the free amino groups regenerated simply by allowing the solution to stand at an acidic pH of 2 or less for at least 2 hours at ambient temperature, or overnight at a temperature of 10°C or less. This procedure is advantageously performed after the cleavage reaction.

### 3. S-Sulfonation

After completion of the citraconylation reaction, 1/20 volume of 1 M Tris hydrochloric acid buffer (pH 8 to 9) is added to the mixture, and the pH is adjusted to 8.6 using glacial acetic acid. 1/100 volume of 2 M aqueous sodium sulphite and 1/100 volume of 30 mM aqueous copper sulphate are added to the resulting mixture, and the mixture is stirred well. The reaction is allowed to go to completion by standing for at least 3 hours at ambient temperature, or overnight at a temperature of 10°C or less. In this condition, the fusion protein is completely solubilised and does not precipitate, even if the urea concentration in the solution is reduced to 3 M or less.

The sulphonate groups introduced at the -SH groups of the cysteine residues by this method can subsequently be removed as follows. 2 mM aqueous reduced glutathione and 1 mM aqueous sodium ethylenediaminediacetate are added to the solution containing the sulphonated peptide. The resulting mixture is then stirred slowly for 2 to 3 hours at ambient temperature and at a pH ranging from about 7.5 to about 9.0.

The above desulphonation reaction is usefully carried out after purification of calcitonin peptide, which typically uses ion exchange column chromatography or high-performance liquid chromatography on a reverse phase column. Disulphide bonds automatically reconstitute once they have been deprotected, forming a bridge between the cysteine residues at positions 1 and 7 of calcitonin.

### 4. Cleavage of the Fusion Protein

In this preferred process, a protease selected from clostripain, Staphylococcus aureus V8 protease and trypsin is used in the cleavage reaction, once the ε-amino groups of the lysine residues have been citraconylated, to obtain calcitonin peptide (CT-Leu) from the fusion protein.

If V8 protease is to be used, the linker should be constructed with a glutamic acid residue in place of arginine. 2 to 3 volumes of distilled water are added to the slurry containing the citraconylated, S-sulphonated fusion protein obtained in a similar manner to that described in 1 above (where the cleavage reaction is to be performed with clostripain or trypsin, 20 mM aqueous calcium nitrate is added, instead of distilled water, to stabilise the enzyme). The urea concentration of the resulting mixture is brought down to 3.3 M, or less, by this dilution. The pH of the mixture is then adjusted with 1 N aqueous sodium hydroxide, 1 N aqueous hydrochloric acid or glacial acetic acid. It is possible to perform the reaction over a pH range of from 7.0 to 9.0, but a pH range of about 7.6 to 8.0 is preferred.

A range of reaction temperatures can be used for the enzyme reaction, from about 20 to about 40°C, but 30°C is preferred. The prepared solution is warmed for 30 minutes, and then the enzyme solution is added, the concentration of the enzyme solution having been suitably adjusted (see below), the volume of the enzyme solution being about 1% of the fusion protein preparation. The cleavage reaction is allowed to proceed for about 2 to 3 hours, after which time the fusion protein is cleaved completely, with the amount of the product, citraconylated, S-sulphonated CT-Leu, reaching equilibrium. The reaction is stopped by adding trifluoroacetic acid to a final concentration of 1.0% w/v. The pH of the reaction mixture, after stopping (the stopped reaction mixture), is 2.0 or less.

Suitable amounts of enzyme are 0.25 to 1.00 unit of clostripain (Sigma), 40 to 200 units of trypsin (Sigma), and 10 to 40 units of V8 protease (Sigma), per mg of fusion protein. As clostripain must be activated by a reducing agent, it should be prepared in advance as a solution containing about 2.5% v/v β-mercaptoethanol. β-Mercaptoethanol should also be added, to a similar final concentration, to the cleavage reaction mixture.

The amount of citraconylated, S-sulphonated CT-Leu obtained by the above process can be measured using high-performance liquid chromatography, performed on a reverse phase column.

Because the pH of the stopped cleavage reaction mixture is 2.0 or less, decitraconylation can be effected simply by allowing the mixture to stand for at least 2 hours at ambient temperature, or overnight at a temperature of 10°C or less. If a precipitate is observed in the reaction mixture following the decitraconylation reaction, it can be removed by centrifugation. S-sulphonated CT-Leu is contained in the resulting supernatant, and can be purified by ion exchange column chromatography or high-performance liquid chromatography on a reverse phase column.

The present invention will now be further illustrated by the following Examples. It will be understood that the Examples are not limiting on the present invention, which should be construed in accordance with the accompanying claims. Where standard techniques or methods are referred to herein, or where no method is specifically mentioned, then, in the absence of any indication to the contrary, suitable procedures can be found in "Molecular Cloning, A Laboratory Manual", Ed's Sambrook, Fritsch and Maniatis, Cold Spring Harbor Laboratory Press, 2nd edition (1989).

### EXAMPLE 1

### Preparation of Human Calcitonin

### A. Preparation of Human Calcitonin Fusion Protein

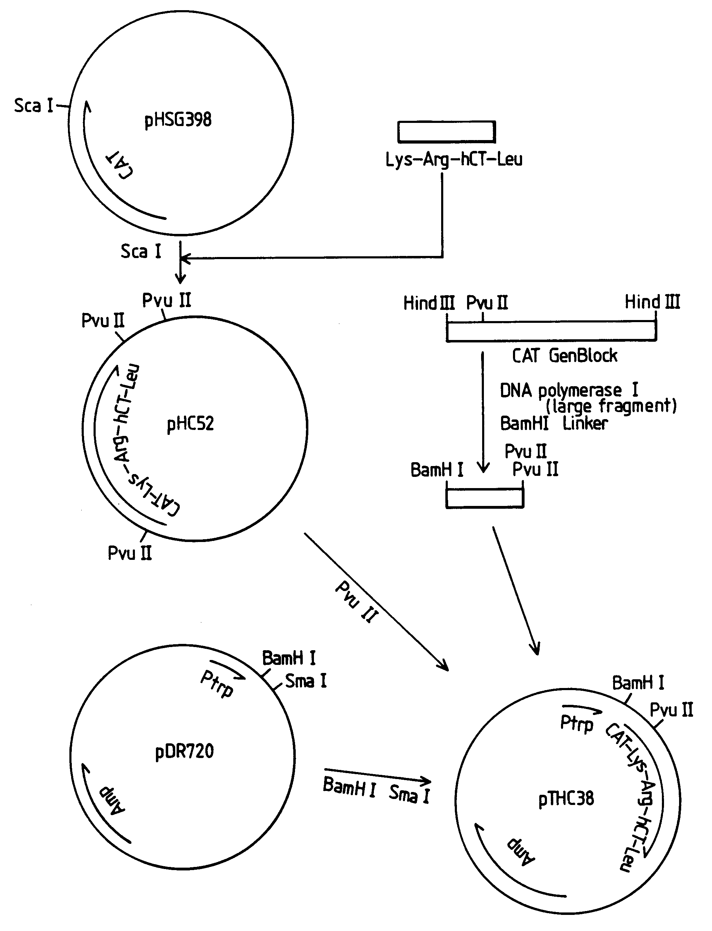

An expression plasmid for a human calcitonin/ chloramphenicol acetyl transferase fusion protein was constructed as follows, and as is shown in the accompanying Figure 1.

An expression plasmid, pHSG 398 [TAKARA: Gene (1987), 61, 63] was used. DNA encoding the peptide sequence Lys-Arg-hCT-Leu, constructed using standard procedures, was inserted at the Sca I site of pHSG 398, the region of the plasmid encoding chloramphenicol acetyl transferase. The resulting plasmid was designated pHC 52.

pHC 52 was then digested with Pvu II and a DNA fragment encoding the carboxy terminal of chloramphenicol acetyl transferase together with the Lys-Arg-hCT-Leu sequence was obtained.

Chloramphenicol acetyl transferase Genblock (Pharmacia) was digested with Hind III and the ends were blunted using DNA polymerase I (large fragment). A BamH I linker was then added and the resulting DNA was digested with Pvu II. A BamH I-Pvu II DNA fragment encoding the amino terminal portion of chloramphenicol acetyl transferase was obtained.

The two DNA fragments obtained above were ligated into the plasmid pDR720 [Pharmacia: Gene (1872), 20, 231] which had been digested with BamH I and Sma I. The resulting expression plasmid was designated pTHC38. In this plasmid, expression of the fusion protein (CAT-Lys-Arg-hCT-Leu) is under the control of a Trp promoter.

Escherichia coli HB101 (Funakoshi Pharmaceutical Co., Ltd.) was transformed with pTHC38 by standard techniques. The transformed Escherichia coli was then cultured under standard conditions at 37°C in a 200 liter tank until an OD₆₀₀ (optical density at 600 nm) of 15 to 20 was attained. 3-Indoleacrylic acid was then added as an inducing agent to a final concentration of 20 µg/ml, and culturing was continued for a further 5 hours.

165 liters of the culture solution obtained by the above process were then subjected to acid treatment by lowering the pH to 3.0, using concentrated sulphuric acid, maintaining the pH at 3.0 for 30 minutes, and then restoring the pH to 7.0 with 5 N aqueous sodium hydroxide. The bacteria were then collected by continuous centrifugation (BTPX-205, manufactured by α-Laval Company).

35.5 liters of the collected bacteria were passed through a homogeniser (Gorlin 15 M, APV Co.) to homogenise the cells. 50 mM Tris acetic acid buffer (pH 7.8), containing 0.5% v/v Triton X-100, was added to the homogenate, to a final quantity of 177.5 liters, to wash the preparation. After washing, the insoluble fraction was collected by centrifugation as above, and 18.8 liters of a concentrated slurry containing the fusion protein were obtained. The concentration of protein in this slurry was 52.1 mg/ml, of which 9.8 mg/ml was fusion protein (representing 1.2 mg/ml human calcitonin peptide), giving a ratio of fusion protein to slurry protein of 18.8%.

The amount of fusion protein in the slurry was determined as follows.

First, amino acid analysis using the Hitachi Model 8500 amino acid analyser was performed on slurry protein hydrolysate. The hydrolysate was prepared by treating the slurry with 6 N aqueous hydrochloric acid at 110°C for 24 hours. The sum of the total amounts of amino acids detected was determined and taken as being representative of total slurry protein. Next, 13% w/v polyacrylamide-sodium dodecyl sulphate electrophoresis was performed on the slurry protein, and protein bands were detected using a densitometer. The ratio of the area value of the fusion protein band (molecular weight 29 340) to that of the total protein band was then determined, and used to calculate the total amount of fusion protein. Human calcitonin peptide could be determined from the proportion of the molecular weight of human calcitonin peptide compared to the molecular weight of the uncleaved fusion protein (12%: 3 520/29 340).

The slurry was then treated to obtain human calcitonin peptide in three ways, one using only urea as the solubilising agent (comparative preparation), the second using both urea and citraconylation, and the third using urea, citraconylation and S-sulphonation.

### 1. Comparative Preparation of Human Calcitonin

Two volumes of 10 M aqueous urea solution were added to 25 ml of slurry obtained in A. The protein was not completely solubilised by this addition. 2 ml of 20 mM aqueous calcium nitrate solution were added to 2 ml of the urea-containing slurry (the urea concentration thereby dropping to 3.3 M), followed by addition of β-mercaptoethanol to a final concentration of 2.5% v/v. The pH of the resulting mixture was then adjusted to 7.8 using glacial acetic acid. The cleavage reaction was then carried out by the addition of activated clostripain solution (calculated to provide 0.5 unit of enzyme to 1 mg of protein) to the mixture at 37°C with stirring. After 2 hours, the reaction was stopped by the addition of trifluoroacetic acid to a final concentration of 1% w/v.

The stopped reaction mixture was then centrifuged and 100 µl of the supernatant obtained thereby was applied to a reverse phase high-performance liquid chromatographic column [TSK (trade mark of Tosoh Co., Ltd.) gel ODS-120T, 4.6 x 250 mm] equilibrated with 20% v/v aqueous acetonitrile containing 0.1% w/v trifluoroacetic acid. The peptides on the column were then eluted at a flow rate of 1.0 ml/minute, increasing acetonitrile concentration linearly from 20 to 30% (from 0 to 5 minutes), 30 to 40% (from 5 to 15 minutes) and 40 to 50% (from 15 to 20 minutes). By monitoring absorbance of the eluates at 225 nm, it was observed that human calcitonin peptide (hCT-Leu) eluted at 15.5 minutes. The production yield (cleavage reaction yield) was calculated at approximately 25% by comparing the yield of hCT-Leu with respect to initial substrate (fusion protein) from the area value on the chromatogram with respect to a standard.

### 2. Preparation of Citraconylated Human Calcitonin

Two volumes of 10 M aqueous urea solution were added to 25 ml of the slurry obtained in A above. 250 µl of citraconic anhydride were added to the resulting mixture in six portions, with stirring at room temperature, in order to effect citraconylation. The citraconic anhydride was added at time intervals of 10 to 15 minutes, and the pH of the reaction solution was maintained at 8.5 to 9.0 by adding 5 N aqueous sodium hydroxide.

The cleavage reaction was carried out on 2 ml of the citraconylated slurry using clostripain, as described in 1 above. The stopped reaction mixture (which had a pH of 1.5) was left to stand overnight at 4°C in order to decitraconylate the protein. The decitraconylated mixture was then centrifuged. 100 µl of the resulting supernatant were then subjected to high-performance liquid chromatography using a reverse phase column (TSK gel ODS-120T, 4.6 x 250 mm), which was prepared and eluted as described in 1 above. Human calcitonin peptide eluted at 15.3 minutes. The cleavage reaction yield was found to be approximately 80%.

The cleavage reaction was then repeated using trypsin instead of clostripain.

A similar procedure to that for clostripain was used. 2 ml of 20 mM aqueous calcium nitrate solution was added to 2 ml of citraconylated slurry. β-Mercaptoethanol was then added to the resulting mixture to a final concentration of 2.5% v/v, and the pH of the mixture was adjusted to 7.8. Trypsin solution (prepared so as to provide 45 units of enzyme to 1 mg of protein) was added to the mixture and the cleavage reaction proceeded with stirring at 37°C. When the reaction was stopped, the cleavage reaction yield was found to be approximately 80%, thereby demonstrating the equivalence of clostripain and trypsin, when used under these circumstances, for the purposes of the present invention.

### 3. Preparation of Citraconylated and S-Sulfonated hCT-Leu

1/20 volume of 1 M Tris acetic acid buffer (pH 8.6), 1/100 volume of 2 M aqueous sodium sulphite solution and 1/1000 volume of 30 mM aqueous copper sulphate solution were all added to 2 ml of citraconylated slurry prepared as in 2 above. The resulting mixture was left to stand overnight at 4°C to sulphonate the protein. The protein was completely solubilised after this procedure.

Cleavage with trypsin was then performed on the citraconylated, S-sulphonated slurry.

2 ml of 20 mM aqueous calcium nitrate solution was added to about 2 ml of the citraconylated and S-sulphonated slurry, and the pH of the mixture was adjusted to 7.8 using 1 N aqueous sodium hydroxide. Trypsin solution (calculated to provide 45 units of enzyme to 1 mg of protein) was added to the resulting solution and the reaction was carried out at 37°C for 2 hours. The reaction was stopped by addition of trifluoroacetic acid to a final concentration of 1% w/v, and the stopped reaction solution was allowed to stand overnight at 4°C. Subsequent analysis using high-performance liquid chromatography, performed as described in 1 above, determined that the yield of the cleavage reaction was 95%.

### EXAMPLE 2

Human calcitonin peptide was prepared by cleaving the human calcitonin peptide fusion protein prepared in Example 1 with trypsin.

700 ml of 10 M aqueous urea solution was added to 350 ml of of the slurry containing fusion protein prepared in Example 1A (total protein: 18.2 g, total fusion protein: 3.4 g, human calcitonin peptide: 411 mg). Citraconylation was performed on the resulting mixture by adding 3 ml of citraconic anhydride in six portions at ambient temperature with stirring. The time intervals between additions of citraconic anhydride were 10 to 15 minutes. The pH of the reaction solution was maintained at 8.5 to 9.0 by the appropriate addition of 5 N aqueous sodium hydroxide during the reaction.

50 ml of 1 M Tris acetic acid buffer (pH 8.6), 10 ml of 2 M aqueous sodium sulphite solution and 1 ml of 30 mM aqueous copper sulphate solution were added to the resulting solution after completion of the citraconylation reaction, and S-sulphonation was effected by allowing the mixture to stand overnight at 4°C.

1 Liter of a 20 mM aqueous solution of calcium nitrate was added to 1 liter of the resulting citraconylated and S-sulphonated reaction solution (containing the equivalent of 388 mg of human calcitonin peptide) and the pH of the mixture was adjusted to 7.8 using glacial acetic acid. The cleavage reaction was then carried out at 37°C for 2 hours by the addition of trypsin solution containing 136 x 10³ enzyme units (40 units of enzyme per 1 mg of fusion protein). Trifluoroacetic acid was added to a final concentration of 1% w/v to the reaction solution, which was then allowed to stand overnight. The reaction mixture was then centrifuged, and the supernatant collected. 100 µl of the supernatant was subjected to analysis by high performance liquid chromatography in a similar manner to that described in Example 1, and the yield of the cleavage reaction was found to be 78%.

Separately, 40 ml (1/50 volume) of the supernatant was applied to a reverse phase high-performance liquid chromatographic column (TSK gel ODS-120T, 55 x 300 mm) equilibrated with 25.2% v/v aqueous acetonitrile containing 0.1% w/v trifluoroacetic acid. The column was then eluted at a flow rate of 30 ml/min using a linear gradient starting at 25.2% (from 0 to 5 minutes) and 25.2 to 44.8% (from 10 to 25 minutes) of acetonitrile concentration. The absorbance of the eluates was monitored at 225 nm, and the fraction that eluted at a retention time of 58.5 minutes (the time at which an S-sulphonated hCT-Leu standard elutes) was collected. Amino acid analysis was performed on the hydrolysate of this fraction, which was prepared by treatment with 6 N aqueous hydrochloric acid at 110°C for 24 hours. The amino acid composition corresponded to that of human calcitonin peptide (hCT-Leu). The amount of peptide collected was 5.1 mg, and the yield was 66.3%.

### EXAMPLE 3

### Large-Scale Preparation of hCT-Leu

7.4 Liters of a 10 M aqueous solution of urea was added, with stirring, to 3.7 liters of the slurry containing fusion protein prepared in Example 1A (total protein: 193 g, total fusion protein: 36.3 g, amount of human calcitonin peptide: 4.4 g). 35 ml of citraconic anhydride was then added to the resulting mixture in seven portions at intervals of 20 minutes. The mixture was maintained at a temperature of 30°C with stirring, and the pH was kept in the range of 8.5 to 9.0 using 5 N aqueous sodium hydroxide.

S-Sulphonation was then performed by adding: 0.5 liter of 1 M Tris acetic acid buffer (pH 8.6); 0.1 liter of 2 M aqueous sodium sulphite solution; and 10 ml of 30 mM aqueous copper sulphate solution, with stirring, to 13.4 liters of the solution obtained above, after completion of the citraconylation reaction. The resulting mixture was allowed to stand overnight at 4°C.

7 Liters of 30 mM aqueous calcium nitrate solution were added to 14 liters of the citraconylated and S-sulphonated reaction solution, and the pH of the mixture was adjusted to 7.8 using glacial acetic acid. The cleavage reaction was carried out at 37°C for 2 hours after the addition of 69 ml of trypsin solution (10 mg/ml, 7.3 x 10⁶ enzyme units). The reaction was stopped by adding 210 ml of trifluoroacetic acid to a final concentration of 1% w/v, and the stopped reaction solution was allowed to stand overnight at 4°C. The solution was then subjected to ultrafiltration (filters from Amicon Co., Ltd.) and 22 liters of a clear liquid were obtained. 100 µl of this clear liquid were applied to a reverse phase high-performance liquid chromatographic column [YMC-ODS (trade mark YMC Co., Ltd.) AM312, 6 x 150 mm] equilibrated with 0.1% w/v aqueous trifluoroacetic acid containing 26% v/v acetonitrile. The column was then eluted at a flow rate of 1.0 ml/min with linearly increasing acetonitrile concentration between 26 and 33% (from 0 to 16 minutes). Monitoring the absorbance of eluants at 225 nm, S-sulphonated human calcitonin peptide eluted at a retention time of 13.3 minutes. The yield of the cleavage reaction was calculated to be 57.1% from the area value on the chromatogram with respect to a standard sample.

The remainder of the above-mentioned clear liquid was applied to a reverse phase high-performance liquid chromatographic preparative column (YMC-ODS-1050-50SRM100) equilibrated with 15% v/v aqueous acetonitrile containing 1% w/v trifluoroacetic acid. The column was eluted at a flow rate of 400 ml/min with a linear gradient of acetonitrile concentration of 15% (from 0 to 10 minutes), 21 to 31% (from 10.1 to 30 minutes), 31% (from 30 to 50 minutes) and 31 to 46% (from 50 to 80 minutes). Fractions were detected in the eluates by monitoring absorption at 225 nm.

The presence of S-sulphonated hCT-Leu was monitored for each fraction using analytical high-performance liquid chromatography as described above. It was determined that S-sulphonated human calcitonin peptide eluted at 40 to 44 minutes, and this fraction was collected. 1.6 liters of the fraction containing S-sulphonated human calcitonin peptide were diluted with distilled water to bring the concentration of S-sulphonated hCT-Leu to 0.2 mg/ml.

Reduced glutathione (to a final concentration of 1 mM) and sodium ethylenediaminediacetate (to a final concentration of 2 mM), respectively, were added to this fraction with adjustment of the pH to 8.5 with 29% v/v aqueous ammonia. After once again adjusting the pH of the mixture to 8.5, desulphonation was allowed to proceed for 3 hours at ambient temperature with slow stirring. After completion of the reaction, the reaction mixture was run a further time on a high-performance liquid preparative column chromatograph under the conditions described above. The fraction containing hCT-Leu was collected. The fraction was concentrated by evaporation under reduced pressure and was then lyophilised to obtain 1.6 g of hCT-Leu as a powder, yield 36.8%. The resulting powder had a purity of 97.3%.

## Claims

1. A process for recovering a peptide from a fusion protein incorporating the peptide, the process comprising the steps of:
i) at least partially solubilising the fusion protein,
ii) subjecting the solubilised fusion protein to proteolytic cleavage and,
iii) isolating the peptide,
characterised in that step i) comprises citraconylating free amino groups of the fusion protein.

2. A process according to Claim 1, wherein step i) additionally comprises, where appropriate, sulphonating free -SH groups or disulphide linkages of the fusion protein.

3. A process according to Claim 1 or 2, wherein the fusion protein is obtained by culturing a host transformed with an expression vector encoding the fusion protein.

4. A process according to Claim 3, wherein the host is Escherichia coli.

5. A process according to Claim 3 or 4, wherein the fusion protein forms insoluble inclusion bodies within the host.

6. A process according to Claim 5, wherein the fusion protein is collected by disruption of the cells followed by washing and centrifugation.

7. A process according to any preceding Claim, wherein the peptide is a calcitonin, a mutant, variant or other modification of a calcitonin possessing calcitonin activity, or a precursor of any of the foregoing.

8. A process according to Claim 7, wherein the peptide is selected from human, chicken, pig, cow, sheep, rat, eel and salmon calcitonins.

9. A process according to Claim 8, wherein the peptide is human calcitonin.

10. A process according to any preceding Claim, comprising amidating the C-terminal of the peptide.

11. A process according to Claim 10, wherein the C-terminal of the peptide is a proline residue.

12. A process according to any preceding claim, wherein the cleavage of step ii) is such as to leave a leucyl group attached to the C-terminal of the peptide, and wherein, optionally, one or more further amino acid residues, not including leucyl, are attached to the leucyl, their number not exceeding 5.

13. A process according to Claim 12, wherein the leucyl group is attached to the carboxyl group of a proline residue, and digesting the resulting peptide with carboxypeptidase Y, so as to yield a peptide having an amidated C-terminal proline residue.

14. A process according to any preceding Claim, wherein solubilisation comprises treatment of the fusion protein with at least one solubilising agent.

15. A process according to Claim 14, wherein the solubilising agent is a solution of urea and/or guanidine hydrochloride.

16. A process according to Claim 14 or 15, wherein the solubilising agent is used in concentrated form.

17. A process according to Claim 16, wherein the solubilising agent is used in a concentration of at least 5 M, and at least two volumes of a preparation of the agent are added to a preparation of the fusion protein.

18. A process according to Claim 17, wherein the solubilising agent is a solution of urea and/or guanidine hydrochloride having a concentration of 8 to 10 M and 5 to 6 M, respectively.

19. A process according to any of Claims 14 to 18, wherein citraconylation of free amino groups is effected after treatment with the solubilisation agent.

20. A process according to Claim 19, wherein free -SH groups or disulphide linkages of the fusion protein are sulphonated after citraconylation of free amino groups.

21. A process according to any preceding Claim, wherein citraconylation is achieved by the addition of a citraconylating agent to a preparation of fusion protein.

22. A process according to Claim 21, wherein the citraconylating agent is citraconic anhydride.

23. A process according to Claim 21 or 22, wherein the citraconylating agent is added to the preparation in several portions, with stirring, at intervals of 5 to 20 minutes.

24. A process according to any preceding Claim, wherein the citraconylation is effective to citraconylate about 95% of free amino groups.

25. A process according to any of Claims 2 to 24, wherein S-sulphonation is achieved by addition of 1/100 volume of 2 M aqueous sodium sulphite and 1/100 volume of 30 mM aqueous copper sulphate to a preparation of the fusion protein, the preparation then being allowed to stand for a period suitable to allow S-sulphonation to go substantially to completion.

26. A process according to any preceding Claim, wherein, after step i), the at least partially solubilised fusion protein is diluted in order to permit a protease to be used in step ii).

27. A process according to any preceding Claim, wherein, after step ii), free amino groups are regenerated.

28. A process according to Claim 27, wherein regeneration is effected by allowing the product of step ii) to stand under acidic conditions.

29. A process according to any preceding Claim where free -SH groups or disulphide linkages have been sulphonated, wherein free -SH groups or disulphide linkages are regenerated after step ii).

30. A process according to any preceding Claim, wherein a protease is used for the proteolytic cleavage of step ii).

31. A process according to Claim 30, wherein the protease is trypsin, clostripain or Staphylococcus aureus V8 protease.

32. A process according to any preceding Claim, wherein the fusion protein comprises at least one secondary peptide component fused with the peptide, the fusion protein having been expressed by a host, and wherein the secondary peptide component is homologous to the host.

33. A process according to any preceding Claim, wherein the fusion protein comprises at least one secondary peptide component fused with the peptide, and which is selected from β-galactosidase, chloramphenicol acetyl transferase, maltose-binding protein and Staphylococcus aureus Protein A.

34. A process according to any preceding Claim, wherein the fusion protein, in addition to the peptide, further comprises at least one secondary peptide component and an amino acid linker sequence linking the peptide and the secondary peptide component.

35. A process according to Claim 34, wherein the linker sequence, either individually or together with one or other of the peptide and secondary peptide component, provides a site for proteolytic cleavage of the fusion protein by a protease.

36. A process according to Claim 35, wherein the protease is trypsin, clostripain and/or Staphylococcus aureus V8 protease.

37. A process according to any preceding Claim, wherein the fusion protein is obtained by culturing a host transformed with an expression vector encoding the fusion protein, the host being Escherichia coli, and the vector being selected from pBR 322, PUC 18 and pDR 720.

38. A process according to any preceding Claim, wherein the fusion protein is expressed by a host selected from Escherichia coli strains HB101, RB791, JM109, JA221 and DH5.

39. A process according to any preceding Claim, wherein trypsin is used in step ii).

40. A process for recovering calcitonin from a fusion protein incorporating calcitonin, the process comprising the steps of:
i) at least partially solubilising the fusion protein,
ii) subjecting the solubilised fusion protein to proteolytic cleavage and,
iii) isolating calcitonin,
wherein step i) comprises citraconylating free amino groups of the fusion protein and, subsequently, where appropriate,
sulphonating free -SH groups or disulphide linkages of the fusion protein,
the fusion protein being at least partially solubilised by treatment with highly concentrated urea and/or guanidine hydrochloride prior to citraconylating the amino groups,
the process further comprising, after sulphonating the free -SH groups, diluting the solubilised fusion protein sufficiently to permit proteolytic action by a protease selected from trypsin, clostripain and Staphylococcus aureus V8 protease.

41. A process for the preparation of calcitonin comprising the steps of:
a) transforming Escherichia coli with an expression vector encoding a fusion protein which incorporates calcitonin, the expression vector enabling the Escherichia coli to express the fusion protein;
b) culturing the transformed Escherichia coli under conditions such that the transformed Escherichia coli expresses the fusion protein as inclusion bodies;
c) disrupting and washing the cultured, transformed Escherichia coli to form a preparation, and harvesting the inclusion bodies from the preparation by centrifugation;
d) at least partially solubilising the fusion protein contained in the inclusion bodies by treatment with a volumetric excess of an aqueous solution of guanidine hydrochloride at a concentration of between 5 and 6 M, and/or by treatment with an aqueous solution of urea at a concentration of between 8 and 10 M to provide a slurry containing at least partially solubilised fusion protein;
e) treating the slurry with an amount of citraconic anhydride in an at least 20 molar excess over the free amino groups thereby to citraconylate the free amino groups;
f) further treating the slurry with at least one agent effective to sulphonate free -SH groups or disulphide linkages present in the fusion protein;
g) diluting the slurry with a diluent effective to reduce the total concentration of guanidine hydrochloride and/or urea to a level at which trypsin is able to function as a protease;
h) adding an amount of trypsin to the diluted slurry sufficient to cleave into parts the fusion protein;
i) isolating a cleaved part of the fusion protein which comprises calcitonin; and, optionally,
removing sulphonate groups and/or restoring free amino groups before or after step i).

42. A peptide when obtained by a process according to any preceding Claim.

43. A calcitonin when obtained by a process according to any of Claims 1 to 41.

44. Human calcitonin according to Claim 43.
